# EUROPEAN PATENT SPECIFICATION

(11) **EP 3 053 562 B1**
(45) Date of publication and mention of the grant of the patent: **02.03.2022**
(21) Application number: 14820632.9
(22) Date of filing: 15.07.2014
(51) Int. Cl.: A61F 13/496, A61F 13/49

(54) **DISPOSABLE PANTS-TYPE DIAPER**
HOSENÄHNLICHE EINWEGWINDEL
COUCHE POUR INCONTINENCE DE TYPE CULOTTE JETABLE

(30) Priority: 30.09.2013 JP 2013205644
(43) Date of publication of application: 10.08.2016
(73) Proprietor: Unicharm Corporation, Ehime 799-0111 (JP)
(72) Inventor: KURITA, Noriyuki, Kanonji-shi Kagawa 769-1602 (JP); MUKAI, Hirotomo, Kanonji-shi Kagawa 769-1602 (JP)
(74) Representative: Staeger & Sperling Partnerschaftsgesellschaft mbB
(86) International application number: PCT/JP2014/068829
(87) International publication number: WO 2015/002328

(56) References cited:
- WO-A1-2011/039988
- WO-A1-2013/018881
- WO-A1-2013/065620
- JP-A- 2008 043 674
- JP-A- 2011 115 229
- JP-A- 2013 031 536

## Description

### {Technical Field}

The present invention relates to disposable pull-on diapers.

### {Background}

Disposable pull-on diapers having a front panel defining a front waist region, a back panel defining a back waist region and a crotch panel defining a crotch region is conventionally known. The disposable pull-on diapers in which the back panel extends downward in the longitudinal direction of the diaper longer than the front panel so that a surplus portion may be used as the buttocks cover portion partially covering a wearer's buttocks is also known.

For example, JP 2008-508082 A (Patent Literature 1) describes a pull-on diaper as a particular embodiment of disposable pull-on wearing articles. This diaper includes an annular elastic belt consisting of a front belt segment and a back belt segment. A length dimension in the longitudinal direction of a side panel defining a back belt segment is set to be larger than a length dimension in the longitudinal direction of the side panel defining the front belt segment. A rectangular absorbent main body extends between the front belt segment and the back belt segment and both end portions of the absorbent main body are attached to the front belt segment and to the back belt segment, respectively. The back belt segment partially defines buttocks-covers on both lateral sides.

The buttocks-covers include elastic members extending in the transverse direction of the diaper under tension.

A disposable diaper described in JP 2008-161572 A (Patent Literature: 2) is of a pant-type including a front exterior sheet and a back exterior sheet wherein a front end portion of an absorbent main body extending from the back side across a crotch region to the front side to cover the back side, the crotch region and the front side is connected to an interior surface of the front side exterior sheet at a transversely central portion while the back end portion is connected to an interior surface of the back side exterior sheet at a central portion in a width direction. The back exterior sheet has a main body portion and an extension portion extending downward from the main body portion. The extension portion has a transversely central portion overlapping the absorbent main body and buttocks-covering portions extending from both sides of the central portion. To the buttocks-covering portions, a plurality of elastic members extending in the transverse direction of the diaper are secured under tension.

### {Citation List}

### {Patent Literature}

{PTL 1}: JP 2008- 508082 A
{PTL 2}: JP 2008-161572 A

Further prior art in this technical field is disclosed in documents WO2013/018881 A1 and WO 2011/039988A1.

### {Summary}

### (Technical Problem}

In the conventional disposable pull-on diapers, a plurality of elastic members extending in the transverse direction of the diaper are secured under tension to the buttocks-cover portions so that the buttocks-covers may constrict in the transverse direction and a plurality of gathers may appear in the transverse direction when the diapers are put on the wearer's body. In this instance, an outer appearance of the buttocks-cover portions may be different from an outer appearance of the crotch panel usually free from the formation of such gathers.

An object of the present invention is to provide a disposable pull-on diaper improved so that the buttocks-covering portions may be absolutely or almost free from the formation of gathers and the outer appearance of the buttocks-cover portions may come near to the outer appearance of the crotch panel.

### {Solution to Problem}

To solve the problem as described above, the present invention is directed to improve the disposable pull-on diaper having a vertical direction and a body-cross direction, and including a front panel defining a front waist region, a back panel defining a back waist region and a crotch panel defining a crotch region and disposed with a bodily-fluid absorbent core.

The disposable pull-on diaper according to the present invention is arranged according to the features of claim 1. Both lateral portions opposed to each other in the body-cross direction of the back panel are joined to respective lateral portions opposed to each other in the body-cross direction of the front panel and the back panel includes a buttocks-covering portion extending downward in the vertical direction beyond the front panel, wherein none of linear elastic members extending in the body-cross direction under tension is present in the buttocks-covering portion. As viewed in the vertical direction, the crotch panel has a front end portion joined to the front panel and a back end portion joined to the buttocks-covering portion so that a mass of the absorbent core may be supported by the front panel and the buttocks-covering portion.

### {Advantageous Effects of Invention}

In the disposable pull-on diaper according to one or more embodiments of the present Invention, the buttocks-covering portion is not disposed with the elastic members in the body-cross direction and, in consequence, the mass of the absorbent core in the crotch panel is supported by the buttocks-covering portion and the front panel in the vertical direction of the diaper. In such buttocks-covering portion, there is no possibility that a plurality of gathers may be distributed in the body-cross direction under contraction of the elastic members. In addition, the mass of the absorbent core in the crotch region is supported by the buttocks-covering portion in cooperation with the front panel and consequently the buttocks-covering portion is under the force acting to pull this portion downward. For this reason, the buttocks-covering portion has a smooth appearance similar to that of the crotch panel having none of gathers distributed in the body-cross direction.

### {Brief Description of Drawings}

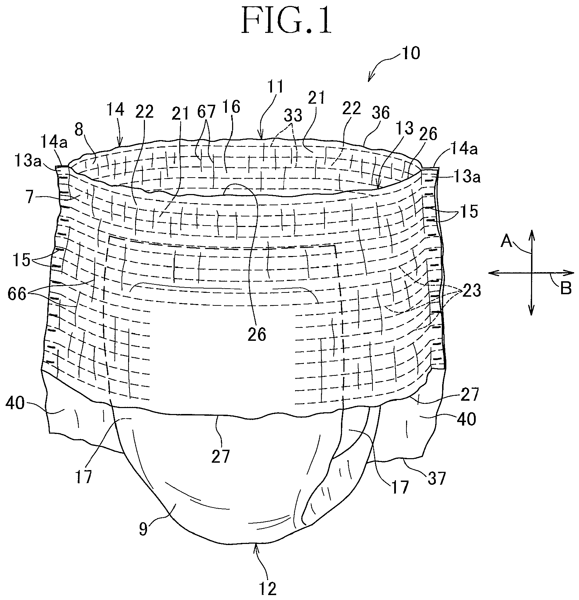

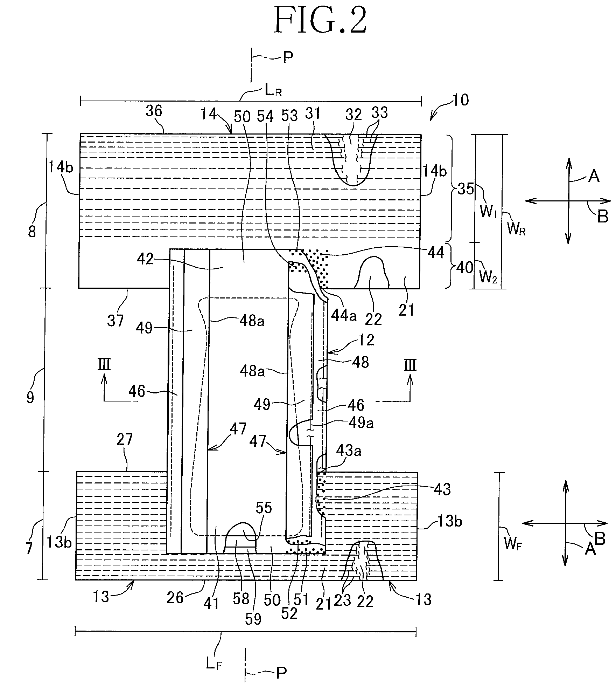

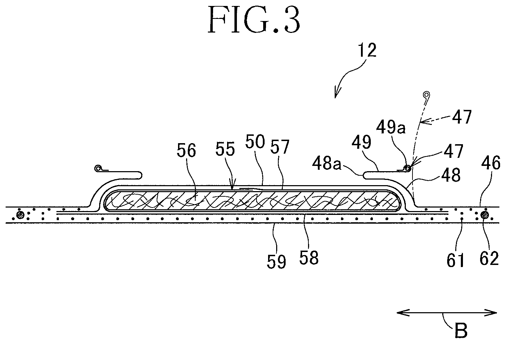

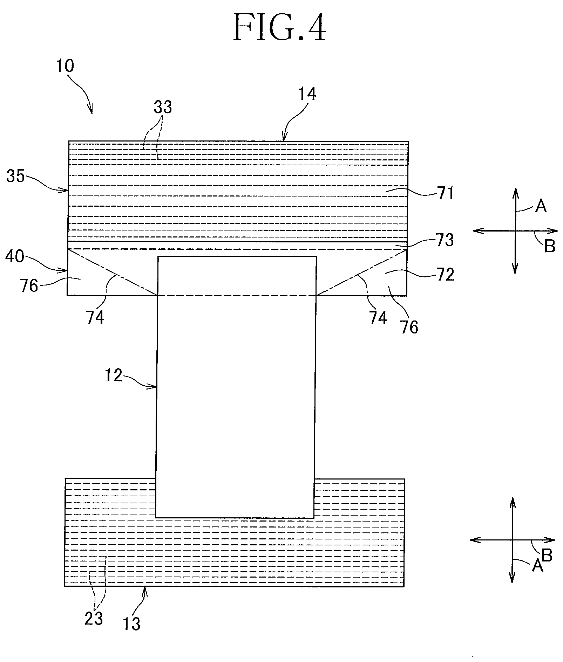

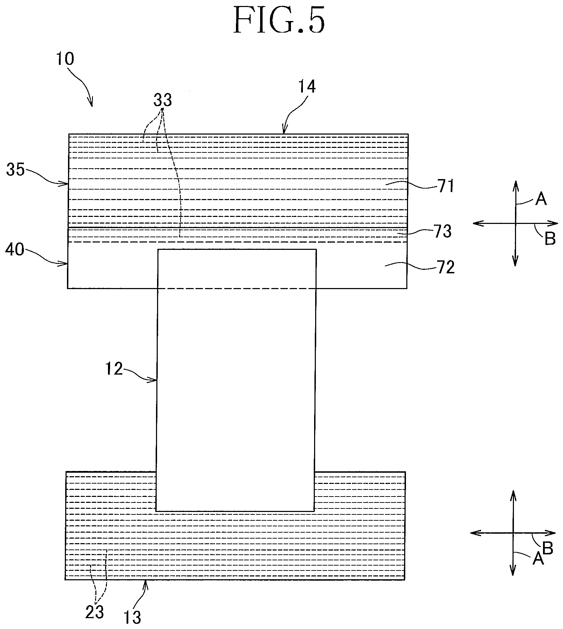

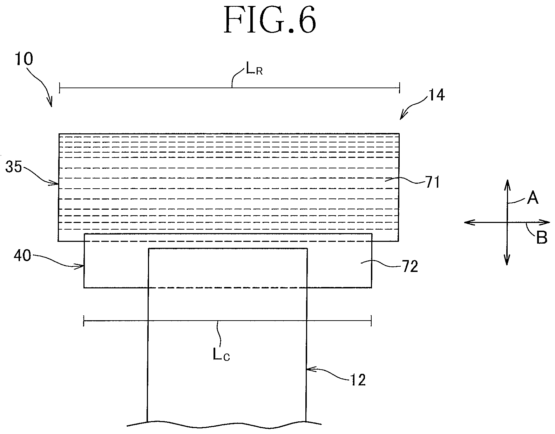

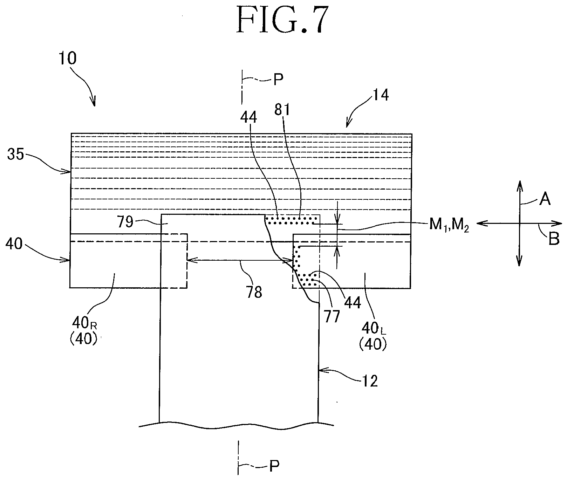

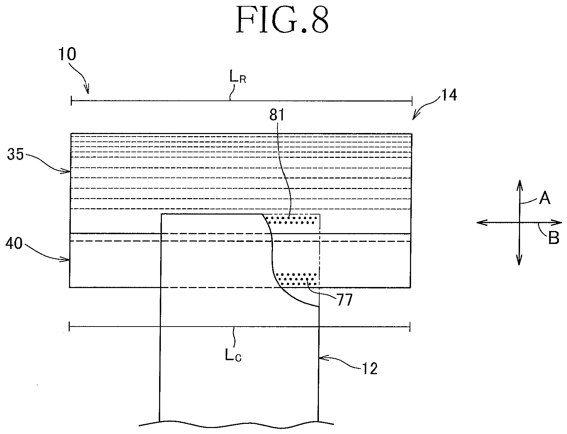

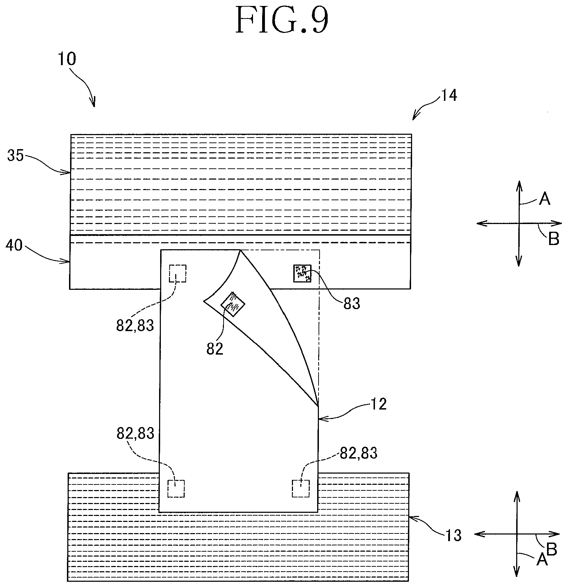

The accompanying drawings illustrate specific embodiments of the present invention including optional and preferred embodiments as well as essential features of the invention.
{Fig. 1} A perspective view of a disposable pull-on diaper.
{Fig. 2} A partially cutaway plan view of the developed diaper of the diaper of Fig. 1.
{Fig. 3} A sectional view taken along line III-III of Fig. 2.
{Fig. 4} A view similar to Fig. 2, illustrating one embodiment.
{Fig. 5} A view similar to Fig. 4, illustrating another embodiment.
{Fig. 6} A partial view similar to Fig. 4, illustrating still another embodiment.
{Fig. 7} A view similar to Fig. 6, illustrating an embodiment according to the present invention.
{Fig. 8} A view similar to Fig. 6, illustrating yet another embodiment.
{Fig. 9} A view similar to Fig. 4, illustrating further another embodiment.

### {Description of Embodiments}

Referring to the accompanying drawings, embodiments of a disposable pull-on diaper are as follows.

The embodiments described below relate to the disposable pull-on diapers illustrated in Figs. 1 through 9 including optional and preferred embodiments as well as essential features of the invention, whereby only Fig. 7 shows an embodiment having all features according to the invention.

Fig. 1 is a perspective view illustrates a diaper 10 as an example of a disposable pull-on diaper. The diaper 10 includes an annular elastic waist panel 11 and a crotch panel 12. The elastic panel 11 includes a front panel 13 defining a front waist region 7 and a back panel 14 defining a back waist region 8. These both panels 13, 14 overlap with each other along respective both lateral edges 13a, 14a and joined to each other along a series of seams 15 arranged in a later defined vertical direction A. The front panel 13 cooperates with the back panel 14 to define a waist-opening 16 and these panels 13, 14 cooperate with the crotch panel 12 to define a pair of leg-openings 17. Part of the crotch panel 12 defines a crotch region 9 (see Fig. 2). In Fig. 1, the vertical direction and a body-cross direction of the diaper 10 are indicated by double-headed arrows A, B.

Fig. 2 is a partially cutaway plan view of the developed diaper 10 of the diaper 10 in Fig. 1, by unjoining the front and back panels 13, 14 along the seams 15 and then planarly extending these panels 13, 14 together with the crotch panel 12.

In the diaper 10, the front panel 13 is formed of an interior sheet 21 and an exterior sheet 22 overlapping with each other and a plurality of elastic members 23 interposed between these interior and exterior sheets 21, 22 so as to extend in parallel under tension in the body-cross direction B. The interior sheet 21 and the exterior sheet 22 are joined to each other, for example, with hot melt adhesive (not shown) and the elastic members 23 are secured to at least one of the interior and exterior sheets 21, 22, for example, with hot melt adhesive (not shown). The front panel 13 has an upper end edge 26, a lower end edge 27 and both lateral edges 13b wherein the upper end edge 26 and the lower end edge 27 respectively have a dimension L_{F} in the body-cross direction B. Both the lateral edges 13b respective have a dimension W_{F} in the vertical direction A (see Fig. 1).

The back panel 14 includes a main region 35 formed of an interior sheet 31 and an exterior 32 overlapping with each other and a plurality of elastic members 33 interposed between these interior and exterior sheets 31, 32 so as to extend in parallel under tension in the body-cross direction B and a buttocks-covering portion 40. This buttocks-covering portion 40 extends downward from the main region 35 and includes the interior sheet 31 and the exterior sheet 32 but none of the elastic members 33 under tension is interposed therebetween. The interior sheet 31 and the exterior sheet 32 are joined to each other, for example, with hot melt adhesive (not shown) wherein the elastic members 33 are secured to at least one of the interior and exterior sheets 31, 32, for example, with hot melt adhesive (not shown). The back panel 14 has an upper end edge 36, a lower end edge 37 and both lateral edges 14b wherein the upper end edge 36 and the lower end edge 37 respective have a dimension L_{R} in the transverse direction L_{R}. Both the lateral edges 14b respectively have a dimension W_{R} in the vertical direction A. The dimension L_{R} is equal to the dimension L_{F} of the front panel 13. The dimension W_{R} is sum of a dimension W₁ of the main region 35 and a dimension W₂ of a buttocks-covering portion 40 wherein the dimension W₁ is equal to the dimension W_{F} in the front panel 13. Hence, as viewed in Fig. 1, the main region 35 having the dimension W₁ overlaps with the front panel 13 and the buttocks-covering portion 40 having the dimension W₂ is positioned lower than the lower end edge 27 of the front panel 13.

The crotch panel 12 has a front end portion 41 and a back end portion 42 wherein the front end portion 41 is joined to the interior surface of the front panel 13, for example, with hot melt adhesive 43. The back end portion 42 is joined to the interior surface of the buttocks-covering portion 40 in the back panel 14, for example, with hot melt adhesive 44. The crotch panel 12 further has both lateral edge portions 46 extending in the vertical direction A and a pair of barrier-flaps 47 respectively extending along both the lateral edge portions 46. The barrier flaps 47 respectively have lower portions 48 extending from the lateral edge portions 46 toward a central line P of the diaper 1 and upper portions 49 folded back along associated inner edges 48a wherein elastic members 49a extending in the vertical direction A are secured under tension but in contractible manner to the respective upper portions 49 (see Fig. 3 also). In the front panel 13, each of the lower portions 48 is joined to an after-mentioned interior sheet 50 of the crotch panel 12, for example, with hot melt adhesive 51 and each of the upper portions 49 is joined to the associated lower portion 48, for example, with hot melt adhesive 52. Of the hot melt adhesive 51 and the hot melt adhesive 52, at least the hot melt adhesive 52 is distributed so that the hot melt adhesive 52 may be located above a lower edge portion 43a in the vertical direction A of the region in which the hot melt adhesive 43 is distributed, i.e., may be located forward in the vertical direction A. In the back panel 14, the lower portion 48 is joined to the interior sheet 50 of the crotch panel 12, for example, with hot melt adhesive 53 and the lower portion 49 is joined to the lower portion 48, for example, with hot melt adhesive 54. Of the hot melt adhesive 53 and the hot melt adhesive 54, at least the hot melt adhesive 54 is distributed so that at least the hot melt adhesive 54 is distributed so that the hot melt adhesive 54 may be located above a lower edge portion 44a in the vertical direction A of the region in which the hot melt adhesive 44 is distributed.

In this manner, the back end portion 42 of the crotch panel 12 is fixed together with the barrier flaps 47 to the back panel 14 above the lower end edge 37 of the back panel 14. For this reason, the lower portions 48 to which the respective upper portions 49 are joined are the lower portions 48 of the portions located defined above the lower end edge 37. In this regard, it is possible in the each of the barrier flaps 47 to join a part of the upper portion 49 to the back panel 14 or to join the lower portion 48 and the upper portion 49 to the back panel 14. The barrier flaps 47 joined to the back panel 14 in such manner advantageously make it easy to pull up the diaper 10 together with the elastic waist panel 11 along the wearer's body with the elastic waist panel 11 pinched by the fingers, thereby making it smooth to put the diaper 10 on the wearer's body.

Fig. 3 is a sectional view taken along line III-III extending the crotch panel 12 in the crotch region 9 in Fig. 2. The crotch panel 12 includes an absorbent core 55 formed of absorptive material 56 such as wood fluff pulp or mixture of the wood fluff pulp and superabsorbent polymer particles and wrapped with wrapping sheet 57 formed of liquid-permeable sheets such as tissue paper, an interior sheet 50 formed of liquid-permeable fibrous nonwoven fabrics or the like and covering at least an interior surface of the absorbent core 55, a liquid-impermeable leakage-barrier sheet 58 formed of liquid-impermeable but vapor-permeable plastic films and covering an exterior surface of the absorbent core 55 and an exterior sheet 59 formed of fibrous nonwoven fabrics or the like ensuring good texture to cover the exterior surface of the leakage-barrier sheet 58. The absorbent core 55 is joined to the interior sheet 50 and to the leakage-barrier sheet 58, for example, with hot melt adhesive (not shown). The leak-barrier sheet 58 and the exterior sheet 59 are joined to each other, for example, with hot melt adhesive 61. The interior sheet 50 and the leakage-barrier sheet 58 extend outward beyond the peripheral edge of the absorbent core 55 so as to overlap with each other and to be joined to each other outside the peripheral edge of the absorbent core 55, for example, with the hot melt adhesive 61. Along the lateral edge portions 46 of the crotch panel 12, the lower portion 48 of the leakage-barrier flap 47 formed of fibrous nonwoven fabrics and the exterior sheet 59 overlap with each other and are joined to each other, for example, with the hot melt adhesive 61. Between the lower portion 48 and the exterior sheet 59 joined to each other, leg elastic members 62 are interposed under tension in the vertical direction A and fixed to at least one of the lower portion 48 and the exterior sheet 59. In the diaper 10 of the state illustrated in Fig. 1 or put on the wearer's body, the elastic member 49a arranged in the upper portion 49 of the barrier-flap 47 constricts and, in consequence, the upper portion 49 folded back on the lower portion 48 as illustrated in Figs. 2 and 3 rises together with the lower portion 48 as indicated by an imaginary line in Fig. 3, i.e., toward the interior side of the diaper 10 and functions to prevent the body exudates from leaking sideways.

In the diaper 10 of the state illustrated in Fig. 1 or put on the wearer's body, the linear elastic members 23, 33 extending in the body-cross direction B in the front panel 13 and the main region 35 of the back panel 14 constrict and, in consequence, a plurality of gathers 66, 67 are formed so as to be distributed in the body-cross direction B in the front panel 13 and the main region 35 of the back panel 14. However, the buttocks-covering portion 40 is free from the formation of such gathers because none of the linear elastic members is arranged in this portion. In this regard, there is a possibility that a small number of gathers may be formed in the buttocks-covering portion 40 also under the influence of the gathers formed in the main region 35. The back end portion 42 of the crotch panel 12 is joined to the buttocks-covering portion 40 so that a mass of the crotch panel 12 may be supported by the front panel 13 and the buttocks-covering portion 40. For this reason, the buttocks-covering portion 40 is under a force acting to pull the buttocks-covering portion 40 down in the vertical direction A. The appearance of the buttocks-covering portion 40 in this state is similar to the appearance of the crotch region 9 in the crotch panel 12 really or substantially free from the gathers distributed in the body-cross direction B. For this reason, the buttocks-covering portion 40 and the crotch region 9 overlapping with each other make it possible for the lower end portion of the back panel 14 to have an appearance which is smooth entirely in the body-cross direction B.

In the diaper 10, as materials for the interior sheet 21 and the exterior sheet 22 in the front panel 13 and the back panel 14, it is possible to use nonwoven fabrics, for example, spunbond nonwoven fabrics or spunbond/meltblown/spunbond nonwoven fabrics etc. each being formed of thermoplastic synthetic fibers having a fineness ranging 1 to 7 dtex and having a mass per unit area ranging 7 to 20 g/m². In the front panel 13 and the back panel 14, the respective interior sheets 21 may be formed of the same nonwoven fabrics or may be formed of different nonwoven fabrics. The respective exterior sheets 22 may be also formed of the same nonwoven fabrics or may be formed of different nonwoven fabrics. It is possible for the interior sheet 21 and/or the exterior sheet 22 to contain elastic thread such as urethane elastic thread so that these sheets 22 may be elastically stretchable. As the elastic members 23, 33, it is possible to use, for example, LYCRA^{(registered trademark)} having a fineness ranging 500 to 700 dtex at a stretch ratio ranging 2 to 3.

In the crotch panel 12, as materials for the interior sheet 50, it is possible to use the nonwoven fabrics formed of thermoplastic synthetic fibers having a fineness ranging 1 to 5 dtex and a mass per unit area ranging 7 to 35 g/m², for example, spunbond nonwoven fabrics or airthrough nonwoven fabrics, if necessary, after having been hydrophilized. As materials for the exterior sheet 59, it is possible to use nonwoven fabrics having a fineness ranging 1 to 7 dtex and a mass per unit area ranging 10 to 30 g/m², for example, spunbond nonwoven fabrics or spunbond/meltblown/spunbond nonwoven fabrics. As materials for the leakage-barrier sheet 58, it is possible to use, for example, plastic films, more preferably, breathable but liquid-impermeable plastic films each having a thickness ranging 0.01 to 0.03 mm. As materials for the elastic members 49a, 62, it is possible to use, for example, LYCRA^{(registered trademark)} having a fineness ranging 500 to 700 dtex at a stretch ratio in a range of 2 to 3.

While the buttocks-covering portion 40 is formed from the interior sheet 21 and the exterior sheet 22 for the diaper 10 illustrated in Fig. 1 (see Fig. 2), it is possible to form the buttocks-covering portion 40 from any one of the interior sheet 21 and the exterior sheet 22. If the interior sheet 21 and the exterior sheet 22 are different from each other with respect to the mass per unit area, the sheet having a relatively large mass per unit area preferably extends downward from the main region 35 to form the buttocks-covering portion 40. The interior sheet 21 and/or the exterior sheet 22 may be formed from a layered sheet of two nonwoven fabric sheets or a layered sheet of fibrous nonwoven fabrics and vapor-permeable plastic films and the buttocks-covering portion 40 also may be formed of such layered sheet.

Fig. 4 is a schematic diagram of the diaper 10 according to an embodiment different from the diaper 10 illustrated in Fig. 1, in a state flatly extended after the seams arranged along both the lateral edges have been disabled. In Fig. 4, the barrier-flaps 47 are not shown. In this diaper 10, the main region 35 and the buttocks-covering portion 40 in the back panel 14 are respectively formed of first and second members 71, 72 and these first and second members 71, 72 are joined to each other in an overlapping sub-region 73 thereof by means of adhesion or welding. The first member 71 defining the main region 35 is made of the same materials as that for the main region 35 in Fig. 2 and an arrangement of the first member 71 is the same as the arrangement of the main region 35 in Fig. 2. The buttocks-covering portion 40 may be formed of the same sheets as the interior sheet 21 and the exterior sheet 22 defining the buttocks-covering portion 40 in Fig. 2 or may be formed of fibrous nonwoven fabrics different from the interior sheet 21 and the exterior sheet 22 in Fig. 2 in thickness and mass per unit area. It is also possible to form the buttocks-covering portion 40 with use of fibrous nonwoven fabrics made of a different kind of thermoplastic synthetic resins from the kind of thermoplastic synthetic resins used for the interior sheet 21 and the exterior sheet 22 in Fig. 2. For example, it is also possible to form the buttocks-covering portion 40 with use of an elastic sheet containing urethane elastic thread. In the diaper 10 having the buttocks-covering portion 40 formed of such elastic sheet, the buttocks-covering portion 40 is substantially free from traces of folding not only in a packaged state but also in a state put on the wearer's body, making it possible to keep the diaper 10 in good appearance. For the buttocks-covering portion 40 in Fig. 4, the buttocks-covering portion 40 may be partially cut off along imaginary lines 74 to change a shape thereof.

Fig. 5 is a schematic diagram similar to Fig. 4, partially illustrating another embodiment of the diaper 10. In this diaper 10, the main region 35 includes the elastic members 33 arranged in the sub-region 73 in which the first member 71 and the second member 72 overlap with each other.

Fig. 6 also is a schematic diagram partially illustrating still another embodiment of the diaper 10 in flatly extended state. Different from the diaper 10 illustrated in Figs. 2 and 4 in which the main region 35 in the back panel 14 and the buttocks-covering portion 40 are the same in the dimension thereof in the body-cross direction B, a dimension L_{Ci} in the body-cross direction B of the buttocks-covering portion 40 is smaller than a dimension L_{R} in the body-cross direction B of the main region 35. The dimension L_{C} of the buttocks-covering portion 40 may be appropriately set according to a size of the buttocks to be covered.

Fig. 7 is a diagram similar to Fig. 6 partially illustrating an embodiment according to the invention of the diaper 10. In this diaper 10, the buttocks-covering portion 40 is bisected in the body-cross direction B to define a right buttock-covering portion 40_{R} and a left buttock-covering portion 40_{L} distanced from and face each other about the center line P. The terms used herein "right" and "left" respectively mean the right and the left for the wearer (not shown) when the diaper 10 is put on the wearer's body. The crotch panel 12 is formed so as to straddle both the right buttock-covering portion 40_{R} and the left buttock-covering portion 40_{L} and to have lower joining regions 77 to be respectively joined to the right buttock-covering portion 40_{R} and the left buttock-covering portion 40_{L}, for example, with the hot melt adhesive 44. In addition, the crotch panel 12 has an extension portion 79 extending upward beyond the buttock-covering portions 40_{R}, 40_{L} and this extension portion 79 is joined, in an upper joining region 81 defined in the upper end portion of the extension portion 7, to the main region 35, for example, with the hot melt adhesive 44. A distance M₂ between the lower joining region 77 and the upper joining region 81 on the crotch panel 12 is larger than a distance M₁ between the lower joining region 77 and the upper joining region 81 on the back panel 13 and, in consequence, the extension portion 79 of the crotch panel 12 necessarily has a sag. For this reason, the mass of the absorbent core 55 (see Fig. 3) in the crotch panel 12 is supported by the front panel 13 and the buttocks-covering portion 40 in the same manner as in the diaper 10 illustrated in Fig. 1. To provide the extension portion 79 with such sag, the crotch panel 12 having, in the extension portion 79 thereof, a region folded in Z-shape and extensible in the vertical direction A may be used.

Fig. 8 is a diagram similar to Fig. 7, illustrating yet another embodiment of the diaper 10, in which the crotch panel 12 has the lower and upper joining region 77, 81 and the buttocks-covering portion 40 is arranged in the same manner as that in Fig. 4, i.e., the dimension L_{C} in the body-cross direction B of the buttocks-covering portion 40 is the same as the dimension L_{R} in the body-cross direction B of the main region 35.

Fig. 9 is a diagram similar to Fig. 4, illustrating further another embodiment. In the diaper 10 illustrated in Fig. 9, hook members 82 of so-called mechanical fastener system are attached to the exterior surface of the crotch panel 12 in required regions and loop members 83 associated with the hook members 82 are attached to the front panel 13 and the buttocks-covering portions 40 in required regions. With use of these members 82, 83, it is possible to attach or to detach the crotch panel 12 to or from the front panel 13 and the buttocks-covering portion 40.

The disclosure relating to the diaper described above may be arranged at least as follows:
A disposable pull-on diaper having a vertical direction and a body-cross direction, and including a front panel defining a front waist region, a back panel defining a back waist region and a crotch panel defining a crotch region and disposed with a bodily-fluid absorbent core, wherein: both lateral portions opposed to each other in the body-cross direction of the back panel are joined to respective lateral portions opposed to each other in the body-cross direction of the front panel and the back panel includes a buttocks-covering portion extending downward in the vertical direction beyond the front panel, wherein none of linear elastic members extending in the body-cross direction under tension is present in the buttocks-covering portion; and, as viewed in the vertical direction, the crotch panel has a front end portion joined to the front panel and a back end portion joined to the buttocks-covering portion so that a mass of the absorbent core is supported by the front panel and the buttocks-covering portion.

The diaper disclosed in the paragraph above may include embodiments at least as described below and these embodiments may be in isolation or in combination of one another.
(1) The back panel includes a main portion in which both the lateral portions are joined to the front panel and the buttocks-covering portion and, of sheets lying interior and exterior sides of the diaper and including the elastic members interposed therebetween, the interior sheet or the exterior sheet extends further downward so as to form the buttocks-covering portion.
(2) The sheets are formed of fibrous nonwoven fabrics, vapor-permeable plastic films or layered sheet of the fibrous nonwoven fabrics and the vapor-permeable plastic films.
(3) The back panel includes a main portion in which both the lateral portions are joined to the lateral portions of the front panel and the buttocks-covering portion and wherein a sheet prepared separately from the main portion, i.e., fibrous nonwoven fabrics, vapor-permeable plastic films or layered sheets of the fibrous nonwoven fabrics and the vapor-permeable plastic films are joined to the main portion, and whereby the buttocks-covering portion is formed.
(4) The buttocks-covering portion extends in the body-cross direction to both the lateral portions of the main portion.
(5) The buttocks-covering portion is bisected about a center line bisecting a dimension between both the lateral portions of the back panel so as to be distanced from and facing each other.
(6) According to the invention, the crotch panel extends upward in the vertical direction beyond the back end portion joined to the buttocks-covering portion and includes a lower joining portion adapted to be joined to the buttocks-covering portion and an upper joining portion adapted to be joined to the main portion and wherein a distance between the lower joining portion and the upper joining portion on the crotch panel is longer than a distance between the lower joining portion and the upper joining portion on the back panel so that a sag may be formed between the lower joining portion and the upper joining portion of the crotch panel.
(7) The buttocks-covering portion is formed of elastically extensible sheets.
(8) The main portion has elastic members under tension in the body-cross direction in a region overlapping with the sheet defining the buttocks-covering portion.
(9) The crotch panel is disposed on both lateral portion as viewed in the body-cross direction with leakage-barriers each of which extends across the crotch region from the front panel to the back panel and has a front end portion overlapping with the front panel and a back end portion overlapping with the back panel wherein, above a lower end edge of the buttocks-covering portion as viewed in the vertical direction of the diaper, the back end portion is fixed to the interior surface of the crotch panel or to the interior surface of the back panel.

### {Reference Signs List}

- 7: front waist region
- 8: back waist region
- 9: crotch region
- 10: pull-on diaper
- 12: crotch panel
- 13: front panel
- 13a: lateral portion
- 14: back panel
- 14a: lateral portion
- 33: elastic members
- 35: main region
- 37: lower end edge
- 40: buttocks-covering portion
- 40_{L}, 40_{R}: buttock-covering portions
- 41: front end portion
- 42: back end portion
- 47: barrier-flaps
- 50: interior sheet
- 55: absorbent core
- 77: lower joining region
- 81: upper joining region
- A: vertical direction
- B: body-cross direction
- M₁, M₂: distance
- P: central line

## Claims

1. A disposable pull-on diaper (1) having a vertical direction (A) and a body-cross direction (B), and including a front panel (13) defining a front waist region (7), a back panel (14) defining a back waist region (14) and a crotch panel (12) defining a crotch region (9) and provided with a bodily-fluid absorbent core (55) extending from the front waist region (7) to the crotch region (9) in the vertical direction (A), the diaper (1) is **characterized in that**:
when the diaper (1) is in the closed configuration, both lateral portions opposed to each other in the body-cross direction (B) of the back panel (14) are joined to respective lateral portions opposed to each other in the body-cross direction (B) of the front panel (13) along a series of seams (15) and the back panel (14) includes an upper end edge (36), an lower end edge (37) and a buttocks-covering portion (40) extending from a lower end edge of the seams (15) to the lower end edge (37) of the back panel (14) in the vertical direction (A) between lateral edges (14b) of the back panel (14) so as to extend downward in the vertical direction (A) beyond the front panel (13), wherein none of linear elastic members extending in the body-cross direction (B) under tension is present in the buttocks-covering portion (40); wherein
the back panel (14) includes a main portion (35) and the buttocks-covering portion (40), the main portion (35) being formed of an interior sheet (31) and an exterior sheet (32) overlapping with each other and a plurality of elastic members (33) extending in parallel under tension in the body-cross direction (B) and interposed between these interior and exterior sheets (31, 32), in which main portion (35) both the lateral portions are joined to the lateral portions of the front panel (13) and wherein, of said sheets (31, 32) lying interior and exterior sides of the diaper (1) including the elastic members (33) interposed therebetween, at least one of the interior sheet (31) and the exterior sheet (32) extends downward from the main portion (35) in the vertical direction (A) so as to form the buttocks-covering portion (40); and
as viewed in the vertical direction (A), the crotch panel (12) has a front end portion (41) joined to the front panel (13) and a back end portion (42) joined to the buttocks-covering portion (40) so that a mass of the absorbent core (55) is supported by the front panel (13) and the buttocks-covering portion (40),
and the crotch panel (12) extends upward in the vertical direction (B) beyond the back end portion (42) joined to the buttocks-covering portion (40) and includes a lower joining portion (77) adapted to be bonded to the buttocks-covering portion (40) and an upper joining portion (81) adapted to be joined to the main portion (35) and
wherein a distance (M2) between the lower joining portion (77) and the upper joining portion (81) on the crotch panel is longer than a distance (M1) between the lower joining portion (77) and the upper joining portion (81) on the back panel (14) so that a sag is formed between the lower joining portion (77) and the upper joining portion (81) of the crotch panel (12).

2. The diaper (1) according to Claim 1, wherein the sheets (31, 32) are formed from fibrous nonwoven fabrics, moisture-permeable plastic films or laminated sheets of the fibrous nonwoven fabrics and the vapor-permeable plastic films.

3. The diaper (1) according to any one of Claims 1 through 2 wherein the buttocks-covering portion (40) extends in the body-cross direction (B) to both the lateral portions of the main portion (35).

4. The diaper (1) according to any one of Claims 1 through 3 wherein the buttocks-covering portion (40) is bisected about a center line (P) bisecting a dimension between both the lateral portions of the back panel (14) so as to be distanced from and facing each other.

5. The diaper (1) according to any one of Claims 1 through 3 wherein the buttocks-covering portion (40) is formed of elastically extensible sheets.

6. The diaper (1) according to any one of Claims 1 through 5 wherein the crotch panel (12) is provided on both lateral portion as viewed in the body-cross direction (B) with leakage-barriers (47) each of which extends across the crotch region (9) from the front panel (13) to the back panel (14) and has a front end portion (41) overlapping with the front panel (13) and a back end portion (42) overlapping with the back panel (14) wherein, above a lower ena edge or tne buttocks-covering portion (4U) as viewed in the vertical direction (A) of the diaper (1), the back end portion (40) is fixed to the interior surface of the crotch panel (12) or 10 the interior surface of the back panel (14).

## Patentansprüche

1. Wegwerf-Schlupfwindel (1), die eine vertikale Richtung (A) und einer Körperquerrichtung (B) aufweist, und mit einen vorderen Einsatz (13), der einen vorderen Taillenbereich (7) definiert, einem hinteren Einsatz (14), der einen hinteren Taillenbereich (14) definiert, und einem Schritteinsatz (12), der einen Schrittbereich (9) definiert und mit einem Körperflüssigkeit absorbierenden Kern (55) versehen ist, der sich von dem vorderen Taillenbereich (7) zu dem Schrittbereich (9) in der vertikalen Richtung (A) erstreckt, wobei die Windel (1) **dadurch gekennzeichnet ist, dass**:
wenn sich die Windel (1) in der geschlossenen Konfiguration befindet, beide seitlichen Abschnitte, die einander in der Körperquerrichtung (B) des hinteren Einsatzes (14) gegenüberliegen, mit jeweiligen seitlichen Abschnitten, die einander in der Körperquerrichtung (B) des vorderen Einsatzes (13) gegenüberliegen, entlang einer Reihe von Nähten (15) verbunden sind, und der hintere Einsatz (14) eine obere Endkante (36), eine untere Endkante (37) und einen das Gesäßabdeckabschnitt (40) umfasst, der sich von einer unteren Endkante der Nähte (15) zu der unteren Endkante (37) des hinteren Einsatzes (14) in der vertikalen Richtung (A) zwischen seitlichen Kanten (14b) des hinteren Einsatzes (14) so erstreckt, dass er sich in der vertikalen Richtung (A) nach unten über den vorderen Einsatz (13) hinaus erstreckt, wobei keines der linearen elastischen Elemente, die sich unter Spannung stehend in der Körperquerrichtung (B) erstrecken, in dem Gesäßabdeckabschnitt (40) vorhanden sind; wobei
der hintere Einsatz (14) einen Hauptabschnitt (35) und den Gesäßabdeckabschnitt (40) umfasst, wobei der Hauptabschnitt (35) aus einer inneren Lage (31) und einer äußeren Lage (32), die einander überlappen, und einer Mehrzahl von elastischen Elementen (33), die sich parallel unter Spannung in der Körperquerrichtung (B) erstrecken und zwischen diesen inneren und äußeren Lagen (31, 32) angeordnet sind, gebildet ist, wobei in dem Hauptabschnitt (35) die beiden seitlichen Abschnitte mit den seitlichen Abschnitten des vorderen Einsatzes (13) verbunden sind und wobei von den Lagen (31, 32), die die Innen- und Außenseiten der Windel (1) mit den dazwischen angeordneten elastischen Elementen (33) bilden, mindestens eine von der inneren Lage (31) und der äußeren Lage (32) sich von dem Hauptabschnitt (35) in der vertikalen Richtung (A) nach unten erstreckt, um den Gesäßabdeckabschnitt (40) zu bilden; und
in der vertikalen Richtung (A) gesehen, der Schritteinsatz (12) einen vorderen Endabschnitt (41), der mit dem vorderen Einsatz (13) verbunden ist, und einen hinteren Endabschnitt (42), der mit dem Gesäßabdeckabschnitt (40) verbunden ist, aufweist, so dass eine Masse des absorbierenden Kerns (55) von dem vorderen Einsatz (13) und dem Gesäßabdeckabschnitt (40) getragen wird,
und sich der Schritteinsatz (12) in vertikaler Richtung (B) über den mit dem Gesäßabdeckabschnitt (40) verbundenen hinteren Endabschnitt (42) hinaus nach oben erstreckt und einen unteren Verbindungsabschnitt (77), der mit dem Gesäßabdeckabschnitt (40) verbunden werden kann, und einen oberen Verbindungsabschnitt (81), der mit dem Hauptabschnitt (35) verbunden werden kann, umfasst, und wobei ein Abstand (M2) zwischen dem unteren Verbindungsabschnitt (77) und dem oberen Verbindungsabschnitt (81) an dem Schritteinsatz länger ist als ein Abstand (M1) zwischen dem unteren Verbindungsabschnitt (77) und dem oberen Verbindungsabschnitt (81) an dem hinteren Einsatz (14), so dass ein Durchhang zwischen dem unteren Verbindungsabschnitt (77) und dem oberen Verbindungsabschnitt (81) des Schritteinsatzes (12) ausgebildet ist.

2. Windel (1) nach Anspruch 1, wobei die Lagen (31, 32) aus Faservliesstoffen, feuchtigkeitsdurchlässigen Kunststofffolien oder laminierten Lagen aus den Faservliesstoffen und den dampfdurchlässigen Kunststofffolien gebildet sind.

3. Windel (1) nach einem der Ansprüche 1 bis 2, wobei sich der Gesäßabdeckabschnitt (40) in Körperquerrichtung (B) zu den beiden seitlichen Abschnitten des Hauptteils (35) erstreckt.

4. Windel (1) nach einem der Ansprüche 1 bis 3, wobei der Gesäßabdeckabschnitt (40) um eine Mittellinie (P) herum halbiert ist, die eine Abmessung zwischen den beiden seitlichen Abschnitten des hinteren Einsatzes (14) so halbiert, dass sie voneinander beabstandet und einander zugewandt sind.

5. Windel (1) nach einem der Ansprüche 1 bis 3, wobei der Gesäßabdeckabschnitt (40) aus elastisch dehnbaren Lagen gebildet ist.

6. Windel (1) nach einem der Ansprüche 1 bis 5, wobei der Schritteinsatz (12) an beiden seitlichen Abschnitten in Körperquerrichtung (B) gesehen mit Auslaufsperren (47) versehen ist, die sich jeweils über den Schrittbereich (9) von dem vorderen Einsatz (13) zu dem hinteren Einsatz (14) erstrecken und einen vorderen Endabschnitt (41), der mit dem vorderen Einsatz (13) überlappt, und einen hinteren Endabschnitt (42), der mit dem hinteren Einsatz (14) überlappt, aufweisen, wobei oberhalb einer unteren Endkante oder des Gesäßabdeckabschnitts (40), in der vertikalen Richtung (A) der Windel (1) gesehen, der hintere Endabschnitt (40) an der Innenfläche des Schritteinsatzes (12) oder an der Innenfläche des hinteren Einsatzes (14) befestigt ist.

## Revendications

1. Couche à enfiler jetable (1) ayant une direction verticale (A) et une direction transversale au corps (B), et comprenant un panneau avant (13) définissant une région de taille avant (7), un panneau arrière (14) définissant une région de taille arrière (14), et un panneau d'entrejambe (12) définissant une région d'entrejambe (9) et comportant un noyau d'absorption de fluides corporels (55) s'étendant de la région de taille avant (7) à la région d'entrejambe (9) dans la direction verticale (A), la couche (1) étant **caractérisée par le fait que** :
lorsque la couche (1) est dans la configuration fermée, les deux parties latérales opposées l'une à l'autre dans la direction transversale au corps (B) du panneau arrière (14) sont réunies à des parties latérales respectives opposées l'une à l'autre dans la direction transversale au corps (B) du panneau avant (13) le long d'une série de coutures (15) et le panneau arrière (14) comprend un bord d'extrémité supérieur (36), un bord d'extrémité inférieur (37) et une partie (40) de recouvrement des fesses, s'étendant d'un bord d'extrémité inférieur des coutures (15) au bord d'extrémité inférieur (37) du panneau arrière (14) dans la direction verticale (A) entre des bords latéraux (14b) du panneau arrière (14) de façon à s'étendre vers le bas dans la direction verticale (A) au-delà du panneau avant (13), aucun élément élastique linéaire s'étendant dans la direction transversale au corps (B) sous tension n'étant présent dans la partie (40) de recouvrement des fesses ; dans laquelle
le panneau arrière (14) comprend une partie principale (35) et la partie (40) de recouvrement des fesses, la partie principale (35) étant formée d'une feuille intérieure (31) et d'une feuille extérieure (32) se chevauchant mutuellement et plusieurs éléments élastiques (33) s'étendant en parallèle sous tension dans la direction transversale au corps (B) et interposés entre ces feuilles intérieure et extérieure (31, 32), partie principale (35) dans laquelle les deux parties latérales sont réunies aux parties latérales du panneau avant (13) et où, parmi lesdites feuilles (31, 32) se trouvant sur des côtés intérieur et extérieur de la couche (1) comprenant les éléments élastiques (33) interposés entre elles, au moins l'une de la feuille intérieure (31) et de la feuille extérieure (32) s'étend vers le bas à partir de la partie principale (35) dans la direction verticale (A) de façon à former la partie (40) de recouvrement des fesses ; et
tel que vu dans la direction verticale (A), le panneau d'entrejambe (12) a une partie d'extrémité avant (41) réunie au panneau avant (13) et une partie d'extrémité arrière (42) réunie à la partie (40) de recouvrement des fesses de telle sorte qu'une masse du noyau absorbant (55) est supportée par le panneau avant (13) et la partie (40) de recouvrement des fesses,
et le panneau d'entrejambe (12) s'étend vers le haut dans la direction verticale (B) au-delà de la partie d'extrémité arrière (42) réunie à la partie (40) de recouvrement des fesses et comprend une partie de liaison inférieure (77) apte à être liée à la partie (40) de recouvrement des fesses et une partie de liaison supérieure (81) apte à être réunie à la partie principale (35), et où une distance (M2) entre la partie de liaison inférieure (77) et la partie de liaison supérieure (81) sur le panneau d'entrejambe est plus longue qu'une distance (M1) entre la partie de liaison inférieure (77) et la partie de liaison supérieure (81) sur le panneau arrière (14) de telle sorte qu'un affaissement est formé entre la partie de liaison inférieure (77) et la partie de liaison supérieure (81) du panneau d'entrejambe (12).

2. Couche (1) selon la revendication 1, dans laquelle les feuilles (31, 32) sont formées à partir de tissus non tissés fibreux, de films plastiques perméables à l'humidité ou de feuilles stratifiées des tissus non tissés fibreux et des films plastiques perméables à la vapeur.

3. Couche (1) selon l'une quelconque des revendications 1 et 2, dans laquelle la partie (40) de recouvrement des fesses s'étend dans la direction transversale au corps (B) jusqu'aux deux parties latérales de la partie principale (35).

4. Couche (1) selon l'une quelconque des revendications 1 à 3, dans laquelle la partie (40) de recouvrement des fesses est partagée en deux autour d'une ligne centrale (P) partageant en deux une dimension entre les deux parties latérales du panneau arrière (14) de façon à être éloignées l'une de l'autre et à se faire mutuellement face.

5. Couche (1) selon l'une quelconque des revendications 1 à 3, dans laquelle la partie (40) de recouvrement des fesses est formée de feuilles élastiquement extensibles.

6. Couche (1) selon l'une quelconque des revendications 1 à 5, dans laquelle le panneau d'entrejambe (12) comporte, sur les deux parties latérales, tel que vu dans la direction transversale au corps (B), des barrières anti-fuites (47) dont chacune s'étend à travers la région d'entrejambe (9) du panneau avant (13) au panneau arrière (14) et a une partie d'extrémité avant (41) chevauchant le panneau avant (13) et une partie d'extrémité arrière (42) chevauchant le panneau arrière (14), où, au-dessus d'un bord d'extrémité inférieur de la partie (40) de recouvrement des fesses, tel que vu dans la direction verticale (A) de la couche (1), la partie d'extrémité arrière (40) est fixée à la surface intérieure du panneau d'entrejambe (12) ou à la surface intérieure du panneau arrière (14).
